(19) Europäisches Patentamt
European Patent Office
Office européen des brevets

(11) **EP 4 810 603 A1**

(12) **EUROPEAN PATENT APPLICATION**
published in accordance with Art. 153(4) EPC

(43) Date of publication:
**23.09.2026 Bulletin 2026/39**

(21) Application number: **24891444.2**

(22) Date of filing: **13.11.2024**

(51) International Patent Classification (IPC):
***C12N 9/04*** (2006.01) ***C12N 1/15*** (2006.01)
***C12N 1/19*** (2006.01) ***C12N 1/21*** (2006.01)
***C12N 5/10*** (2006.01) ***C12N 15/53*** (2006.01)
***C12N 15/63*** (2006.01) ***C12Q 1/32*** (2006.01)

(52) Cooperative Patent Classification (CPC):
**C12N 5/10; C12N 9/0004; C12N 15/63; C12N 15/74; C12N 15/80; C12N 15/81; C12Q 1/32**

(86) International application number:
**PCT/JP2024/040387**

(87) International publication number:
**WO 2025/105408 (22.05.2025 Gazette 2025/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **15.11.2023 JP 2023194701**

(71) Applicant: **Amano Enzyme Inc.**
**Nagoya-shi**
**Aichi 460-8630 (JP)**

(72) Inventor: **ONO, Atsushi**
**Kakamigahara-shi, Gifu 509-0109 (JP)**

(74) Representative: **Müller-Boré & Partner Patentanwälte PartG mbB**
**Friedenheimer Brücke 21**
**80639 München (DE)**

Remarks:
The complete document including Reference Table(s) and the Sequence Listing(s) can be downloaded from the EPO website

# (54) MODIFIED ENZYME

(57) The purpose of the present invention is to provide an HBDH of which the enzymatic activity after being treated at least at one of prescribed temperatures in a temperature range of 40°C or higher is improved compared with the enzymatic activity of an enzyme including the amino acid sequence represented by SEQ ID NO: 1 after being treated at the given temperature. Provided is a modified HBDH including an amino acid sequence having such a structure that a substitution with a specific amino acid residue is introduced into at least any one position selected from position-47, position-48, position-49, position-53, position-54, position-55, position-63, position-81, position-147, position-210, position-240, position-241, position-250, position-251 and position-252 in the amino acid sequence represented by SEQ ID NO: 1. The modified HBDH is improved in terms of HBDH activity after being treated at least at one of prescribed temperatures in a temperature range of 40°C or higher compared with the HBDH activity of an enzyme including the amino acid sequence represented by SEQ ID NO: 1 after being treated at the same temperature.



Processed by Luminess, 75001 PARIS (FR)

## Description

### Technical Field

The present invention relates to a modified enzyme.

### Background Art

D-3-hydroxybutyrate dehydrogenase (HBDH: EC1.1.1.30) belongs to a short-chain dehydrogenase/reductase (SDR) family, and is an enzyme that reversibly catalyzes the oxidation reaction of 3-hydroxybutyric acid to acetoacetic acid using $NAD^+$ as a coenzyme. Hydroxybutyric acid is called a ketone body together with acetoacetic acid and acetone.

A rapid increase in blood ketone body concentration is known as a cause of ketoacidosis (DKA) which is a severe complication of type 1 diabetes. Therefore, it is recommended to monitor the ketone body levels in patients with type 1 diabetes in order to prevent complications. The HBDH is industrially important enzyme used for such ketone body measurement.

The HBDH has been isolated from a variety of microorganisms. For example, PTL 1 discloses an HBDH derived from Rhodobacter sphaeroides, NPL 1 discloses an HBDH derived from Paracoccus denitrificans, NPL 2 discloses an HBDH derived from Ralstonia pickettii T1, NPL 3 discloses an HBDH derived from Mycobacterium phlei ATCC354, NPL 4 discloses an HBDH derived from Staphylococcus xylosus, NPL 5 discloses an HBDH derived from Pseudomonas fragi, NPL 6 discloses an HBDH derived from Alcaligenes faecalis, NPL 7 discloses an HBDH derived from Acidovorax sp. strain SA1, NPL 8 discloses an HBDH derived from Bacillus cereus T, and NPL 9 discloses an HBDH derived from Zoogloea ramigera I-16-M.

While microorganism-derived HBDH is unstable against heat, PTL 2 and PTL 3 each disclose that an HBDH derived from Alcaligenes faecalis and an HBDH derived from Pseudomonas sp. are HBDHs excellent in stable thermal stability even at 37°C.

### Citation List

#### Patent Literature

PTL 1: Japanese Patent Laid-open Publication No. H11-318438
PTL 2: Japanese Patent Laid-open Publication No. H8-70856
PTL 3: Japanese Patent Laid-open Publication No. 2003-339385


#### Non Patent Literature

NPL 1: Biochim. Biophys. Acta 871, 302-309 (1986)
NPL 2: J. Biosci. Bioeng. 101, 501-507 (2006)
NPL 3: J. Gen. Microbiol. 104, 123-126 (1978)
NPL 4: Acta Microbiol. Pol. 43, 33-45 (1994)
NPL 5: J. Mol. Biol. 355, 722-733 (2006)
NPL 6: Acta Crystallogr. Sect. F 65, 331-335 (2009)
NPL 7: J. Biosci. Bioeng. 97, 78-81 (2004)
NPL 8: Can. J. Microbiol. 19, 673-677 (1973)
NPL 9: J. Biochem. 89, 625-635 (1981)


### Summary of Invention

#### Technical Problem

In view of the industrial importance of the HBDH, it is desired to create an HBDH having heat resistance of which the activity after being treated under heating conditions is improved as compared with a wild-type HBDH.

Therefore, an object of the present invention is to provide an HBDH of which the enzymatic activity after being treated at least at one of prescribed temperatures in a temperature range of 40°C or higher is improved as compared with

the enzymatic activity of a wild-type HBDH after being treated at the prescribed temperature. Solution to Problem

The present inventor has attempted to impart heat resistance to enhance the activity after the heat treatment by modifying HBDH derived from Ralstonia pickettii. However, even when many variants were produced, variants having such heat resistance could hardly be obtained. Among them, it has been found by chance that in a small number of variants, in the enzymatic activity after being treated at least at one of prescribed temperatures in a temperature range of 40°C or higher, heat resistance is further improved as compared with the enzymatic activity of a wild-type HBDH after being treated at the prescribed temperature. That is, the present invention provides inventions of the following aspects.

Item 1. A modified enzyme including a polypeptide shown in any one of the following (I) to (III):

(I) a polypeptide consisting of an amino acid sequence obtained by introducing at least one of

[A] a substitution of the amino acid residue at position 47 with a methionine residue,
[B] a substitution of the amino acid residue at position 48 with a histidine residue,
[C] a substitution of the amino acid residue at position 49 with a leucine residue,
[D] a substitution of the amino acid residue at position 53 with a phenylalanine residue, a leucine residue, a proline residue, or a glutamic acid residue,
[E] a substitution of the amino acid residue at position 54 with a leucine residue, an alanine residue, or a phenylalanine residue,
[F] a substitution of the amino acid residue at position 55 with a glycine residue, a glutamic acid residue, a methionine residue, or a phenylalanine residue,
[G] a substitution of the amino acid residue at position 63 with a leucine residue,
[H] a substitution of the amino acid residue at position 81 with an isoleucine residue,
[I] a substitution of the amino acid residue at position 147 with a glycine residue,
[J] a substitution of the amino acid residue at position 210 with a valine residue,
[K] a substitution of the amino acid residue at position 240 with an alanine residue,
[L] a substitution of the amino acid residue at position 241 with a phenylalanine residue,
[M] a substitution of the amino acid residue at position 250 with a valine residue,
[N] a substitution of the amino acid residue at position 251 with a proline residue, and
[O] a substitution of the amino acid residue at position 252 with an isoleucine residue,

into the amino acid sequence set forth as SEQ ID NO: 1;
(II) a polypeptide in which one or several amino acid residues other than the substituted amino acid residues are substituted, added, inserted, or deleted in the amino acid sequence in which the substitution shown in at least one of [A] to [O] is introduced, and the 3-hydroxybutyrate dehydrogenase activity after treatment at least at one of prescribed temperatures in a temperature range of 40°C or higher is improved as compared to the activity of the polypeptide consisting of the amino acid sequence set forth as SEQ ID NO: 1 after treatment at the prescribed temperature; and
(III) a polypeptide in which the sequence identity of regions that do not include the substituted amino acid residues is 70% or more in the amino acid sequence in which the substitution shown in at least one of [A] to [O] is introduced, and the 3-hydroxybutyrate dehydrogenase activity after treatment at least at one of prescribed temperatures in a temperature range of 40°C or higher is improved as compared to the activity of the polypeptide consisting of the amino acid sequence set forth as SEQ ID NO: 1 after treatment at the prescribed temperature.

Item 2. A DNA encoding the modified enzyme described in Item 1.

Item 3. An expression cassette or recombinant vector including the DNA described in Item 2.

Item 4. A transformant obtained by transforming a host using the expression cassette or recombinant vector described in Item 3.

Item 5. A method for producing a modified enzyme, the method including a step of culturing the transformant described in Item 4.

Item 6. An enzyme preparation containing the modified enzyme described in Item 1.

Item 7. A sensor for measuring a ketone body, containing the modified enzyme described in Item 1.

Item 8. A kit for measuring a ketone body, containing the modified enzyme described in Item 1.

Item 9. A method for measuring a ketone body in a sample, including a step of bringing a sample for which a ketone body is to be measured into contact with the modified enzyme under the modified enzyme activity condition described in Item 1.

Advantageous Effects of Invention

According to the present invention, there is provided a modified HBDH of which the enzymatic activity after being

treated at least at one of prescribed temperatures in a temperature range of 40°C or higher is improved as compared with the enzymatic activity of a wild-type HBDH after being treated at the prescribed temperature.

Description of Embodiments

Hereinafter, the present invention will be described in detail. Note that 20 amino acid residues in amino acid sequences may be abbreviated as one-letter codes. That is, glycine (Gly) is G, alanine (Ala) is A, valine (Val) is V, leucine (Leu) is L, isoleucine (Ile) is I, phenylalanine (Phe) is F, tyrosine (Tyr) is Y, tryptophan (Trp) is W, serine (Ser) is S, threonine (Thr) is T, cysteine (Cys) is C, methionine (Met) is M, aspartic acid (Asp) is D, glutamic acid (Glu) is E, asparagine (Asn) is N, glutamine (Gln) is Q, lysine (Lys) is K, arginine (Arg) is R, histidine (His) is H, and proline (Pro) is P.

In the present specification, the term "non-polar amino acid" includes alanine, valine, leucine, isoleucine, proline, methionine, phenylalanine, and tryptophan. The term "non-charged amino acid" includes glycine, serine, threonine, cysteine, tyrosine, asparagine, and glutamine. The term "acidic amino acid" includes aspartic acid and glutamic acid. The term "basic amino acid" includes lysine, arginine, and histidine.

In the present specification, the term "substitution" includes not only a case where a substitution of an amino acid residue is artificially introduced, but also a case where a substitution of an amino acid residue is naturally introduced, that is, a case where amino acid residues are originally different. In the present specification, the substitution of an amino acid residue may be an artificial substitution or a natural substitution, but an artificial substitution is preferred.

1. Modified 3-hydroxybutyrate dehydrogenase (modified HBDH)

A modified enzyme of the present invention, that is, modified 3-hydroxybutyrate dehydrogenase includes a polypeptide shown in any one of the following (I) to (III):

(I) a polypeptide consisting of an amino acid sequence obtained by introducing at least one of

[A] a substitution of the amino acid residue at position 47 with a methionine residue,
[B] a substitution of the amino acid residue at position 48 with a histidine residue,
[C] a substitution of the amino acid residue at position 49 with a leucine residue,
[D] a substitution of the amino acid residue at position 53 with a phenylalanine residue, a leucine residue, a proline residue, or a glutamic acid residue,
[E] a substitution of the amino acid residue at position 54 with a leucine residue, an alanine residue, or a phenylalanine residue,
[F] a substitution of the amino acid residue at position 55 with a glycine residue, a glutamic acid residue, a methionine residue, or a phenylalanine residue,
[G] a substitution of the amino acid residue at position 63 with a leucine residue,
[H] a substitution of the amino acid residue at position 81 with an isoleucine residue,
[I] a substitution of the amino acid residue at position 147 with a glycine residue,
[J] a substitution of the amino acid residue at position 210 with a valine residue,
[K] a substitution of the amino acid residue at position 240 with an alanine residue,
[L] a substitution of the amino acid residue at position 241 with a phenylalanine residue,
[M] a substitution of the amino acid residue at position 250 with a valine residue,
[N] a substitution of the amino acid residue at position 251 with a proline residue, and
[O] a substitution of the amino acid residue at position 252 with an isoleucine residue,

into the amino acid sequence set forth as SEQ ID NO: 1;
(II) a polypeptide in which one or several amino acid residues other than the substituted amino acid residues are substituted, added, inserted, or deleted in the amino acid sequence in which the substitution shown in at least one of [A] to [O] is introduced, and the 3-hydroxybutyrate dehydrogenase activity after treatment at least at one of prescribed temperatures in a temperature range of 40°C or higher is improved as compared to the activity of the polypeptide consisting of the amino acid sequence set forth as SEQ ID NO: 1 after treatment at the prescribed temperature; and
(III) a polypeptide in which the sequence identity of regions that do not include the substituted amino acid residues is 70% or more in the amino acid sequence in which the substitution shown in at least one of [A] to [O] is introduced, and the 3-hydroxybutyrate dehydrogenase activity after treatment at least at one of prescribed temperatures in a temperature range of 40°C or higher is improved as compared to the activity of the polypeptide consisting of the amino acid sequence set forth as SEQ ID NO: 1 after treatment at the prescribed temperature.

The amino acid sequence set forth as SEQ ID NO: 1 is an amino acid sequence of a wild-type 3-hydroxybutyrate

dehydrogenase (HBDH) derived from Ralstonia pickettii.

The polypeptides of (I) to (III) may be polypeptides containing the substitution of any one of [A] to [O] singly (single-substituted forms of HBDH derived from Ralstonia pickettii), or may be polypeptides containing the substitutions [A] to [O] in combination of two or more thereof (multi-substituted forms of HBDH derived from Ralstonia pickettii).

Among the polypeptides of (I) to (III), preferred examples include polypeptides containing the substitutions shown in the column of "Type of substitution" in Table 1 among the substitutions of at least any of [A] to [O]. In Table 1, each preferable substitution may be represented by an amino acid residue position and an amino acid residue after the substitution as shown in the column of "Abbreviation of substitution". A specific sequence of the polypeptide of (I) when having each preferred substitution is shown in the column of "Sequence of polypeptide of (I)" in Table 1.

[Table 1]

| Type of substitution | Abbreviation of substitution | Sequence of polypeptide (I) |
|---|---|---|
| Substitutions of [A], [B], and [C] | 47M/48H/49L | SEQ ID NO: 2 |
| Substitutions of [D], [E], and [F] | 53F/54L/55G | SEQ ID NO: 3 |
| | 53L/54L/55E | SEQ ID NO: 4 |
| | 53P/54A/55M | SEQ ID NO: 5 |
| | 53E/54F/55F | SEQ ID NO: 6 |
| Substitution of [G] | 63L | SEQ ID NO: 7 |
| Substitution of [H] (81I) | 81I | SEQ ID NO: 8 |
| Substitution of [I] (147G) | 147G | SEQ ID NO: 9 |
| Substitution of [J] (210V) | 210V | SEQ ID NO: 10 |
| Substitutions of [K] and [L] | 240A/241F | SEQ ID NO: 11 |
| Substitutions of [M], [N], and [O] | 250V/251P/252I | SEQ ID NO: 12 |

In the polypeptide of (II), amino acid modifications introduced may include any one of the modifications including substitution, addition, insertion, and deletion alone (for example, substitution alone) or include two or more of the modifications (for example, substitution and insertion). The number of amino acid differences in a portion other than the amino acid residue into which the substitution shown in at least one of [A] to [O] is introduced in the polypeptide of (II) may be 1 or several, and is, for example, 1 to 77, 1 to 60, 1 to 50, 1 to 40, or 1 to 30, preferably 1 to 25, 1 to 20, 1 to 15, more preferably 1 to 10, further preferably 1 to 8, 1 to 7, 1 to 6, 1 to 5, or 1 to 4, still more preferably 1 to 3, and particularly preferably 1 or 2, or 1.

The sequence identity of the amino acid in a portion other than the amino acid residue into which the substitution shown in at least one of [A] to [O] is introduced in the polypeptide of (III) with respect to the portion corresponding to the amino acid sequence set forth as SEQ ID NO: 1 may be 70% or more, and is preferably 80% or more, more preferably 85% or more, further preferably 90% or more, still more preferably 95% or more, 96% or more, 97% or more, 98% or more, and particularly preferably 99% or more or 99.5% or more.

In the polypeptide of (III), the "sequence identity" refers to a value of amino acid sequence identity obtained by bl2seq program (Tatiana A. Tatsusova, Thomas L. Madden, FEMS Microbiol. Lett., Vol. 174, p 247-250, 1999) in BLAST PACKAGE [sgi32 bit edition, Version 2.0.12; available from National Center for Biotechnology Information (NCBI)]. Parameter settings may be as follows: Gap insertion Cost value: 11 and Gap extension Cost value: 1.

In the polypeptides of (II) and (III), since the amino acids at position 16 (I), position 154 (Y), position 158 (K), position 184 (P), position 187 (V), and position 189 (T) in the amino acid sequence set forth as SEQ ID NO: 1 are considered to contribute to binding of NAD (nicotinamide adenine dinucleotide), the amino acids at the position 93 (Q), position 141 (S), position 143 (H), position 151 (K), and position 154 (Y) are considered to contribute to binding of a substrate, and all of these amino acids are considered to contribute to 3-hydroxybutyrate dehydrogenase activity, it is desirable not to introduce substitutions or deletions at these sites.

In the polypeptides of (II) and (III), when an amino acid substitution is introduced in the portion other than the amino acid residue into which the substitution shown in at least one of [A] to [O] is introduced, examples of a preferred aspect of the amino acid substitution introduced include a conservative substitution. That is, examples of the conservative substitution in the polypeptides of (II) and (III) include the following substitutions: when an amino acid to be substituted is a non-polar amino acid, a substitution with other non-polar amino acids; when an amino acid to be substituted is a non-charged amino acid, a substitution with other non-charged amino acids; when an amino acid to be substituted is an acidic

amino acid, a substitution with other acidic amino acids; and when an amino acid to be substituted is a basic amino acid, a substitution with other basic amino acids.

The heat resistance, which is "improved as compared with the heat resistance of the polypeptide consisting of the amino acid sequence set forth as SEQ ID NO: 1", of the polypeptides of (II) and (III) refers to a temperature characteristic in which the HBDH activity after treatment at least at one of prescribed temperatures in a temperature range of 40°C or higher is higher than the HBDH activity after treatment at the prescribed temperature of the polypeptide consisting of the amino acid sequence set forth as SEQ ID NO: 1. The improved heat resistance of the polypeptides of (II) and (III) may be recognized at least at one of prescribed temperatures in a temperature range of 40°C or higher (the time to be subjected to the prescribed temperature is 15 minutes), and the temperature range is preferably 45°C or higher. Since the polypeptide consisting of the amino acid sequence set forth as SEQ ID NO: 1 has remarkably deteriorated heat resistance particularly at 50°C or higher, the temperature range is more preferably 50°C or higher. The upper limit of the temperature range is not particularly limited, and is, for example, 60°C or lower and preferably 55°C or lower. Specific examples of the temperature range include 40 to 60°C, 45 to 60°C, 50 to 60°C, or 50 to 55°C.

## 2. DNA

A DNA of the present invention is a DNA encoding "1. Modified 3-hydroxybutyrate dehydrogenase (modified HBDH)" above.

The DNA of the present invention is not particularly limited as long as it is a DNA including a nucleotide sequence encoding a modified HBDH consisting of the polypeptide shown in any of (I) to (III) described in the above "1. Modified 3-hydroxybutyrate dehydrogenase (modified HBDH)". Examples of the nucleotide sequence of DNA encoding the amino acid sequence set forth as SEQ ID NO: 1 (wild-type HBDH derived from Ralstonia pickettii), which is a reference sequence for the polypeptides shown in (I) to (III), include SEQ ID NO: 13. Therefore, the DNA of the present invention can be appropriately designed by those skilled in the art using SEQ ID NO: 13 as a reference sequence.

Examples of the DNA of the present invention include DNAs shown in any one of the following (i) to (iii):

(i) a DNA consisting of a nucleotide sequence obtained by introducing at least one of substitutions [a] to [o] shown in the following table into the nucleotide sequence set forth as SEQ ID NO: 13:

[a] a substitution at positions 139 to 141 with a nucleotide sequence encoding a methionine residue,
[b] a substitution of the amino acid residues at positions 142 to 144 with a nucleotide sequence encoding a histidine residue,
[c] a substitution of the amino acid residues at positions 145 to 147 with a nucleotide sequence encoding a leucine residue,
[d] a substitution of the amino acid residues at positions 157 to 159 with a nucleotide sequence encoding a phenylalanine residue, a leucine residue, a proline residue, or a glutamic acid residue,
[e] a substitution of the amino acid residues at positions 160 to 162 with a nucleotide sequence encoding a leucine residue, an alanine residue, or a phenylalanine residue,
[f] a substitution of the amino acid residues at positions 163 to 165 with a nucleotide sequence encoding a glycine residue, a glutamic acid residue, a methionine residue, or a phenylalanine residue,
[g] a substitution of the amino acid residues at positions 187 to 189 with a nucleotide sequence encoding a leucine residue,
[h] a substitution of the amino acid residues at positions 241 to 243 with a nucleotide sequence encoding an isoleucine residue,
[i] a substitution of the amino acid residues at positions 439 to 441 with a nucleotide sequence encoding a glycine residue,
[j] a substitution of the amino acid residues at positions 628 to 630 with a nucleotide sequence encoding a valine residue,
[k] a substitution of the amino acid residues at positions 718 to 720 with a nucleotide sequence encoding an alanine residue,
[l] a substitution of the amino acid residues at positions 721 to 723 with a nucleotide sequence encoding a phenylalanine residue,
[m] a substitution of the amino acid residues at positions 748 to 750 with a nucleotide sequence encoding a valine residue,
[n] a substitution of the amino acid residues at positions 751 to 753 with a nucleotide sequence encoding a proline residue, and
[o] a substitution of the amino acid residues at positions 754 to 756 with a nucleotide sequence encoding an isoleucine residue;

(ii) a DNA encoding a polypeptide in which the heat resistance is improved as compared to the heat resistance of the polypeptide consisting of the amino acid sequence set forth as SEQ ID NO: 1, the DNA hybridizing under stringent conditions with DNA consisting of a nucleotide sequence complementary to the DNA shown in (i); and
(iii) a DNA encoding a polypeptide in which the heat resistance is improved as compared to the heat resistance of the polypeptide consisting of the amino acid sequence set forth as SEQ ID NO: 1, the DNA having a homology of 70% or more with the DNA shown in (i).

Specific examples of the DNA of (i) above are easily determined by those skilled in the art on the basis of the nucleotide sequence of SEQ ID NO: 13. Specific examples of the nucleotide sequences of the DNAs encoding polypeptides set forth as SEQ ID NOS: 2 to 12 in Table 1 include the nucleotide sequences of SEQ ID NOS: 14 to 24.
For the DNA of (ii) above, the expression "under stringent conditions" refers to conditions of incubating in 6×SSC (1×SSC is 0.15 M NaCl, 0.015 M sodium citrate, pH 7.0) containing 0.5% SDS, 5×Denhartz's [Denhartz's, 0.1% bovine serum albumin (BSA), 0.1% polyvinylpyrrolidone, 0.1% Ficoll 400], and 100 μg/ml of a salmon sperm DNA at 50°C to 65°C for 4 hours to overnight.
The hybridization under stringent conditions is specifically performed by the following method. That is, a nylon membrane on which a DNA library or a cDNA library is immobilized is prepared, and the nylon membrane is blocked at 65°C in a prehybridization solution containing 6×SSC, 0.5% SDS, 5×Denhartz's, and 100 μg/ml of a salmon sperm DNA. Thereafter, each probe labeled with $^{32}P$ is added and incubated at 65°C overnight. This nylon membrane is washed in 6×SSC at room temperature for 10 minutes, in 2×SSC containing 0.1% SDS at room temperature for 10 minutes, and in 0.2×SSC containing 0.1% SDS at 45°C for 30 minutes, then autoradiography is performed, and a DNA specifically hybridized with the probe can be detected.
The homology of the DNA of (iii) above may be 70% or more, and is preferably 80% or more, or 85% or more, more preferably 90% or more or 93% or more, further preferably 95% or more, even more preferably 98% or more, still more preferably 98.5% or more or 99% or more, and particularly preferably 99.3% or more or 99.5% or more.
Here, the "homology" of DNA is calculated using a disclosed or commercially available software with an algorithm that makes a comparison using the reference sequence as a query sequence. Specifically, BLAST, FASTA, or GENETYX (manufactured by GENETYX K.K.), or the like can be used, and these may be used with default parameters being set.
The DNA of the present invention can be obtained, for example, by introducing at least one of the substitutions [a] to [o] into the DNA encoding the polypeptide consisting of the amino acid sequence set forth as SEQ ID NO: 1 (wild-type HBDH derived from Ralstonia pickettii). The DNA of the present invention can also be artificially synthesized by a total gene synthesis method.
In the case of the DNA encoding a polypeptide consisting of the amino acid sequence set forth as SEQ ID NO: 1, from a nucleic acid construct such as a plasmid in which the nucleotide sequence set forth as SEQ ID NO: 13 is incorporated, the nucleotide sequence can be acquired by a conventional method using PCR.
As the method for artificially modifying an amino acid sequence by introducing a mutation into a gene, known methods such as a Kunkel method and a Gapped duplex method, and a mutation introduction kit using a site-directed mutagenesis method, for example, QuikChange™ Site-Directed Mutagenesis Kit (Stratagene), GeneTailor™ Site-Directed Mutagenesis System (Invitrogen), TaKaRa Site-Directed Mutagenesis System (Mutan-K, Mutan-Super Express Km, etc.: Takara Bio Inc.), and the like can be used.
The DNA of the present invention includes various kinds of DNA derived from codon degeneracy. Artificial production of various kinds of DNA encoding the same amino acid sequence can be easily performed using a known genetic engineering method. For example, in genetic engineering production of protein, the expression level of a protein of interest may be low when the frequency of use of a codon used on an original gene encoding the protein is low in the host. In this case, high expression of the protein of interest can be achieved by optimizing the codon usage frequency for the host without changing the encoded amino acid sequence.
As an index representing the codon usage frequency, the total host optimal codon usage frequency of each codon may be adopted. The optimal codon is defined as a codon having the highest usage frequency among codons corresponding to the same amino acid. The codon usage frequency is not particularly limited as long as it is optimized for the host, and examples of the optimal codon for Escherichia coli include the following. F: phenylalanine (ttt), L: leucine (ctg), I: isoleucine (att), M: methionine (atg), V: valine (gtg), Y: tyrosine (tat), stop codon (taa), H: histidine (cat), Q: glutamine (cag), N: asparagine (aat), K: lysine (aaa), D: aspartic acid (gat), E: glutamic acid (gaa), S: serine (agc), P: proline (ccg), T: threonine (acc), A: alanine (gcg), C: cysteine (tgc), W: tryptophan (tgg), R: arginine (cgc), G: glycine (ggc).
The nucleotide sequence of DNA in which a mutation is introduced into a nucleotide sequence can be confirmed by sequencing by a conventional method. Specific examples of the sequencing method include a dideoxynucleotide chain termination method (Sanger et al. (1977) Proc. Natl. Acad. Sci. USA 74: 5463), and a sequence analysis method using an appropriate DNA sequencer. Examples of the method for confirming whether DNA is DNA encoding a polypeptide of interest include a method in which a sequenced nucleotide sequence is compared with an unsubstituted nucleotide sequence such as the nucleotide sequence set forth as SEQ ID NO: 13, and a method in which an amino acid sequence

deduced from the sequenced nucleotide sequence is compared with an unsubstituted amino acid sequence such as the amino acid sequence set forth as SEQ ID NO: 1.

3. Expression cassette or recombinant vector

An expression cassette or recombinant vector of the present invention includes the DNA of the present invention described in the above "2. DNA". The expression cassette or recombinant vector of the present invention can be obtained by linking a promoter and a terminator to the DNA of the present invention, or by inserting the expression cassette of the present invention or the DNA of the present invention into an expression vector.

The expression cassette of the present invention or the recombinant vector of the present invention may further include, as control factors, transcription elements such as an enhancer, a CCAAT box, a TATA box, and an SPI site, in addition to promoter and a terminator. These control factors may be operably linked to the DNA of the present invention. The expression "operably linked" means that various control factors that control the DNA of the present invention are linked to the DNA of the present invention in a state of being capable of operating in a host cell.

Regarding the recombinant vector of the present invention, as the expression vector, those constructed for recombination from phage, plasmid, or virus capable of autonomously growing in the host are suitable. Such expression vectors are well-known, and examples of a commercially available expression vector include pQE vector (QIAGEN GmbH), pTrc99A, pDR540, and pRIT2T (GE Healthcare Bio-Sciences KK), and pET vector (Merck KGaA, Darmstadt, Germany). The expression vector may be used by selecting an appropriate combination with a host cell, and for example, in the case of using Escherichia coli as a host cell, a combination of the pET vector and an Escherichia coli strain DH5$\alpha$, a combination of the pET vector and an Escherichia coli strain BL21 (DE3), a combination of the pDR540 vector and an Escherichia coli strain JM109, or the like is mentioned.

4. Transformant

A transformant of the present invention is obtained by transforming a host with the expression cassette or recombinant vector of the present invention described in the above "3. Expression cassette or recombinant vector".

The host used for the production of the transformant of the present invention is not particularly limited as long as the gene can be introduced, the expression cassette or recombinant vector is stable and capable of autonomous growth, and a trait of a gene including the DNA of the present invention can be expressed, but for example, bacteria belonging to the genus Escherichia such as Escherichia coli, the genus Bacillus such as Bacillus subtilis, the genus Pseudomonas such as Pseudomonas putida, and the genus Chryseobacterium such as Chryseobacterium proteolyticum,; yeasts, and the like are preferably exemplified, and other than, the host may be an animal cells, an insect cell, a plant cell, and the like.

The transformant of the present invention can be obtained by introducing the expression cassette of the present invention or the recombinant vector of the present invention into a host. The place where the DNA of the present invention is introduced is also not particularly limited as long as a target gene can be expressed, and may be on a plasmid or on a genome. Specific examples of the method for introducing the expression cassette of the present invention or the recombinant vector of the present invention include a recombinant vector method and a genome editing method. Conditions for introducing the expression cassette or the recombinant vector into the host may be appropriately set according to the type of the host, and the like. When the host is a bacterium, for example, a method using a competent cell by a calcium ion treatment, an electroporation method, and the like are mentioned. When the host is a yeast, for example, an electroporation method, a spheroplast method, a lithium acetate method, and the like are mentioned. When the host is an animal cell, for example, an electroporation method, a calcium phosphate method, a lipofection method, and the like are mentioned. When the host is an insect cell, for example, a calcium phosphate method, a lipofection method, an electroporation method, and the like are mentioned. When the host is a plant cell, for example, an electroporation method, an Agrobacterium method, a particle gun method, a PEG method, and the like are mentioned.

Whether or not the expression cassette of the present invention or the recombinant vector of the present invention has been incorporated into a host can be confirmed by a PCR method, a Southern hybridization method, a Northern hybridization method, and the like.

When it is confirmed whether or not the expression cassette of the present invention or the recombinant vector of the present invention has been incorporated into a host by a PCR method, for example, the genome DNA, the expression cassette, or the recombinant vector may be separated and purified from a transformant.

For example, when the host is a bacterium, the separation and purification of the expression cassette or the recombinant vector are performed based on a lysate obtained by lysing the bacterium. As a lysis method, for example, a treatment is performed with a lytic enzyme such as lysozyme, and as necessary, a protease, other enzymes, and a surfactant such as sodium lauryl sulfate (SDS) are used in combination.

Physical crushing methods such as freeze thawing and French press processing may be combined. The separation and purification of a DNA from a lysate can be performed, for example, by appropriately combining a

deproteinization treatment using a phenol treatment and a protease treatment, a ribonuclease treatment, an alcohol precipitation treatment, and a commercially available kit.

DNA cleavage can be performed according to a conventional method, for example, using a restriction enzyme treatment. As a restriction enzyme, for example, a type II restriction enzyme that acts on a specific nucleotide sequence is used. Binding between the DNA and the expression cassette or the expression vector is performed, for example, using a DNA ligase.

Thereafter, PCR is performed by designing a primer specific to the DNA of the present invention using the separated and purified DNA as a template. The amplification product obtained by PCR is subjected to agarose gel electrophoresis, polyacrylamide gel electrophoresis, capillary electrophoresis, or the like, and is stained with ethidium bromide, SYBR Green solution, and the like, and then the amplification product is detected as a band, so that transformation can be confirmed.

PCR can be performed using a primer labeled with a fluorescent dye or the like in advance to detect an amplification product. A method of binding an amplification product to a solid phase such as a microplate and confirming the amplification product by fluorescence, an enzyme reaction, or the like may also be adopted.

5. Method for producing modified 3-hydroxybutyrate dehydrogenase (modified HBDH)

A method for producing a modified 3-hydroxybutyrate dehydrogenase of the present invention is a method for producing the enzyme described in the above "1. Modified 3-hydroxybutyrate dehydrogenase (modified HBDH)", the method including a step of culturing the transformant of the present invention. Note that, when one of the substitutions [A] to [O] contained in the modified HBDH is naturally introduced, the modified HBDH can be obtained by a production method including a step of culturing a microorganism producing the modified HBDH.

The culture conditions may be appropriately set in consideration of the nutritional physiological properties of the transformant or the microorganism, and liquid culture is preferably mentioned. In the case of performing industrial production, ventilating/stirring culture is preferred. As a nutrient source of the medium, those required for growth of the transformant or the microorganism can be used. A carbon source may be any assimilable carbon compound, and examples thereof include glucose, sucrose, lactose, maltose, molasses, and pyruvic acid. A nitrogen source may be any assimilable nitrogen compound, and examples thereof include peptone, meat extract, yeast extract, casein hydrolysate, and soybean cake alkaline extract. In addition to the carbon source and the nitrogen source, for example, phosphates, carbonates, sulfates, salts of magnesium, calcium, potassium, iron, manganese, zinc, and the like, specific amino acids, specific vitamins, and the like may be used as necessary.

The culture temperature can be appropriately set as long as the transformant of the present invention or the microorganisms can grow, and the transformant or the microorganisms produce a modified HBDH, and is preferably about 15 to 45°C and more preferably about 25 to 39°C. The culturing may be completed at an appropriate time with reference to the time when the modified HBDH reaches the highest yield, and the culturing time is usually about 12 to 48 hours.

After the transformant or the microorganisms are cultured, culture supernatant and/or the bacterial cells can be recovered by subjecting the culture broth to a separation method such as centrifugation. The bacterial cells are treated by a mechanical method such as ultrasonic wave and French press or a lytic enzyme such as lysozyme, and as necessary, are solubilized by using an enzyme such as protease or a surfactant such as sodium lauryl sulfate (SDS), whereby a water-soluble fraction containing a predetermined modified HBDH can be obtained. By selecting an appropriate expression cassette or expression vector and a host, the expressed modified HBDH can also be secreted into the culture broth.

The water-soluble fraction containing the modified HBDH obtained as described above may be subjected to a purification treatment as it is, or may be subjected to a purification treatment after the modified HBDH in the water-soluble fraction is concentrated. The concentration can be performed by, for example, concentration under reduced pressure, membrane concentration, a salting-out treatment, fractionation precipitation with a hydrophilic organic solvent (for example, methanol, ethanol, and/or acetone), or the like.

The purification treatment of the modified HBDH can be performed, for example, by appropriately combining methods such as gel filtration, adsorption chromatography, ion exchange chromatography, and affinity chromatography. The purified modified HBDH may be powderized by lyophilization, vacuum drying, spray drying, or the like if necessary.

6. Enzyme preparation

The modified HBDH can be provided in a form of an enzyme preparation. Therefore, the present invention also provides an enzyme preparation containing the modified HBDH described in the above "1. Modified 3-hydroxybutyrate dehydrogenase (modified HBDH)" as an active ingredient.

The content of the modified HBDH in the enzyme preparation of the present invention is not particularly limited as long as the activity of the modified HBDH as an active ingredient is exhibited when the enzyme preparation is used.

The enzyme preparation of the present invention may or may not contain other components, which are other than

the modified HBDH, as long as the effect of the present invention can be obtained. Examples of the other components include enzymes other than the modified HBDH, additives, and culture residues generated by the production method.

Examples of the other enzymes include amylase ($\alpha$-amylase, $\beta$-amylase, or glucoamylase), glucosidase ($\alpha$-glucosidase or $\beta$-glucosidase), galactosidase ($\alpha$-galactosidase or $\beta$-galactosidase), protease (acidic protease, neutral protease, or alkaline protease), peptidase (leucine peptidase or aminopeptidase), lipase, esterase, cellulase, phosphatase (acid phosphatase or alkaline phosphatase), nuclease, deaminase, oxidase, dehydrogenase (other than the modified HBDH), glutaminase, pectinase, catalase, dextranase, transglutaminase, protein deamidase, and pullulanase. These other enzymes may be contained singly or in combination of a plurality of kinds thereof.

Examples of the additives include an excipient, a buffer agent, a suspending agent, a stabilizer, a preservative, an antiseptic, and physiological saline. Examples of the excipient include starch, dextrin, maltose, trehalose, lactose, D-glucose, sorbitol, D-mannitol, sucrose, and glycerol. Examples of the buffer agent include phosphate, citrate, and acetate. Examples of the stabilizer include propylene glycol and ascorbic acid. Examples of the preservative include phenol, benzalkonium chloride, benzyl alcohol, chlorobutanol, and methylparaben. Examples of the antiseptic include ethanol, benzalkonium chloride, para-hydroxybenzoic acid, and chlorobutanol. These additives may be contained singly or in combination of a plurality of kinds thereof.

Examples of the culture residues include components derived from a culture medium, extraneous proteins, and bacterial cell components.

The form of the enzyme preparation of the present invention is not particularly limited, and examples thereof include a liquid form and a solid form (such as powder or granules). The enzyme preparation of the above form can be prepared by a generally known method.

The use application of the enzyme preparation of the present invention is not particularly limited as long as it is generally a use application utilizing the activity of HBDH. Preferred use applications include ketone body measurement. In this use application, a ketone body to be measured is 3-hydroxybutyric acid (R-3-hydroxybutyric acid). The ketone body measurement will be described in detail in "9. Method for measuring ketone body in sample".

## 7. Sensor for measuring ketone body

The modified HBDH can be used as a sensing means in a sensor for measuring a ketone body. Therefore, the present invention also provides a sensor for measuring a ketone body, the sensor containing the modified HBDH described in the above "1. Modified 3-hydroxybutyrate dehydrogenase (modified HBDH)".

The sensor for measuring a ketone body of the present invention includes the above-described modified HBDH, and can be configured as an object that converts a change based on the reaction of 3-hydroxybutyric acid (R-3-hydroxybutyric acid) with $NAD^+$ (nicotinamide adenine dinucleotide) and/or a derivative thereof by the modified HBDH and an amount thereof into a readable signal.

Specific examples of the change based on the reaction of 3-hydroxybutyric acid (R-3-hydroxybutyric acid) with $NAD^+$ and/or a derivative thereof by the modified HBDH include consumption of 3-hydroxybutyric acid and a change in redox state of $NAD^+$ and/or a derivative thereof.

Examples of the signal capable of reading the change based on the reaction of 3-hydroxybutyric acid (R-3-hydroxybutyric acid) with $NAD^+$ and/or a derivative thereof by the modified HBDH and the amount thereof include an electrochemical signal and an optical signal.

Examples of the electrochemical signal include a signal obtained by converting a chemical state (for example, the presence of 3-hydroxybutyric acid or the like) into an electrical signal (for example, current or the like). Examples of the optical signal include absorption and/or emission based on redox state of $NAD^+$ and/or a derivative thereof (for example, the presence of $NAD^+$ and/or a derivative thereof and/or NADH and/or a derivative thereof).

In the sensor for measuring a ketone body of the present invention, the modified HBDH is preferably immobilized on an insoluble support. The immobilization mode of the modified HBDH is not particularly limited, and examples thereof include physical immobilization by adhesion, adsorption, absorption, swelling, and the like, chemical or biochemical immobilization by specific non-covalent bonding, and chemical immobilization by covalent bonding.

In the modified HBDH, the modified HBDH alone may be immobilized on the insoluble support, or the modified HBDH may be immobilized on the insoluble support in a form of a composition together with other components. A specific aspect when the modified HBDH is immobilized on the insoluble support in a form of a composition includes an aspect in which the composition is physically immobilized (preferably immobilized by adhesion) on the insoluble support.

At least the surface of the insoluble support is composed of a solid or a solid material which does not dissolve in the reaction system between the modified HBDH and 3-hydroxybutyric acid and can immobilize the modified HBDH. Specific examples of the material constituting at least the surface of the insoluble support include a swellable material (for example, gelatin or the like), a porous substrate (for example, cellulose, filter paper, or the like), a resin, glass, a redox polymer (that is, a polymer having a redox mediator bonded thereto), a metal, and carbon.

The shape of an insoluble carrier is not particularly limited, and examples thereof include a substrate shape, a

flake shape, a stick shape, and a bead shape.

Preferred examples of the insoluble carrier include an electrode whose surface is composed of the above material. The modified HBDH is preferably physically immobilized on such an electrode, and more preferably, the composition containing the modified HBDH is physically immobilized (preferably immobilized by adhesion).

A specific aspect of the sensor for measuring a ketone body of the present invention may be any form employed as a biosensor, and examples thereof include a sensor chip, a microtiter plate, a test strip, and an electrochemical flow cell.

8. Kit for measuring ketone body

A kit for measuring a ketone body of the present invention includes an enzyme preparation used for measuring a ketone body among the enzyme preparations described in the above "6. Enzyme preparation" (hereinafter, also referred to as "enzyme preparation for measuring a ketone body") or the sensor for measuring a ketone body described in the above "7. Sensor for measuring ketone body".

The kit for measuring a ketone body of the present invention may further include other appropriate items used for measurement and/or collection of a sample, in addition to the enzyme preparation for measuring a ketone body or the sensor for measuring a ketone body.

Examples of such other items include a coenzyme, a reduction reagent, a redox mediator, a buffer solution, a fluorescent probe, a deproteinization liquid, and a lancet device. The kit for measuring a ketone body of the present invention may contain these other items singly or in combination of two or more kinds thereof.

Examples of the coenzyme include $NAD^+$ (nicotinamide adenine dinucleotide), an $NAD^+$ derivative (for example, nicotinamide adenine dinucleotide phosphate ($NADP^+$), thionicotinamide-NAD, pyridine aldehyde-NAD, acetylpyridine-NAD, carba-NAD, or the like), and $FAD^+$ (flavin adenine dinucleotide). These coenzymes may be used singly or in combination of a plurality of kinds thereof. Among these coenzymes, $NAD^+$ and $NADP^+$ are preferable.

Examples of the reduction reagent include Nitrotetrazolium Blue and Tetrazolium Blue. These reduction reagents may be used singly or in combination of a plurality of kinds thereof.

Examples of the redox mediator include 1-methoxy-5-methylphenazinium methyl sulfur, 1-methoxy-phenazine methosulfate, 1-methoxy-phenazinium ethyl sulfate, Thiol-reactive PES, and Amine-reactive PES. These redox mediators may be used singly or in combination of a plurality of kinds thereof. Among these redox mediators, 1-methoxy-5-methylphenazinium methyl sulfur is preferable.

Examples of the buffer solution include an acetate buffer solution, a phosphate buffer solution, a triethanolamine buffer solution, a tris-hydrochloric acid buffer solution, and a GOOD buffer solution. Examples of the GOOD buffer solution include PIPES, MES, and MOPS. These buffer solutions may be used singly or in combination of a plurality of kinds thereof. Among these buffer solutions, a GOOD buffer solution is preferable, and PIPES is more preferable.

9. Method for measuring ketone body in sample

A method for measuring a ketone body in a sample of the present invention includes a step of bringing a sample for which a ketone body is to be measured into contact with the modified HBDH described in the above "1. Modified 3-hydroxybutyrate dehydrogenase (HBDH)" under the 3-hydroxybutyrate dehydrogenase activity condition.

The method for measuring a ketone body in a sample of the present invention is performed on the basis of the following reaction catalyzed by the modified HBDH.

[Chemical Formula 1] (R)-3-hydroxybutyric acid + $NAD^+$ $\Leftrightarrow$ acetoacetic acid + NADH + $H^+$

In a specific aspect of the method for measuring a ketone body in a sample of the present invention, 3-hydroxybutyric acid in the sample is quantified. In a more specific aspect, the method for measuring a ketone body in a sample of the present invention includes (a) a step of bringing a sample containing 3-hydroxybutyric acid into contact with the modified HBDH; (b) a step of reacting 3-hydroxybutyric acid in the sample with $NAD^+$ (nicotinamide adenine dinucleotide) and/or a derivative thereof by the modified HBDH; and (c) a step of detecting or quantifying a change based on the reaction of 3-hydroxybutyric acid (R-3-hydroxybutyric acid) with $NAD^+$ and/or a derivative thereof. A inactivation step of the modified HBDH may be further included after the step (b) and before the step (c).

In the step (a), the sample is not particularly limited as long as it is a sample in which a ketone body is to be measured, and examples thereof include a biological sample derived from a subject in which a ketone body is to be measured. Examples of the subject in which a ketone body is to be measured include mammals such as humans. Examples of the biological sample include body fluids, excretions, and tissues, and specific examples thereof include blood samples (whole blood, serum, plasma), urine, semen, prostate fluid, tears, saliva, sweat, ascites, cerebrospinal fluid, milk, lymph, and tissue extraction samples.

In the step (a), the modified HBDH can be used so that the amount of the modified HBDH used per 1 g of 3-

hydroxybutyric acid in the sample is, for example, 0.1 μg or more, preferably 1.0 μg or more, more preferably 5.0 μg or more, further preferably 10 μg or more, and particularly preferably about 15 μg or more. The upper limit of the used amount is also not particularly limited, and is, for example, 10000 μg or less, preferably 1000 μg or less, more preferably 500 μg or less, further preferably 100 μg or less, and particularly preferably 50 μg or less per 1 g of 3-hydroxybutyric acid in the sample. These used amounts can be determined through a preliminary experiment.

In order to bring the sample into contact with the modified HBDH, a coexisting system of the sample and the modified HBDH may be constructed. For example, the coexisting system of the sample and the modified HBDH can be constructed, for example, by (a1) preparing a mixed solution of a sample and an enzyme preparation used for measuring a ketone body among the enzyme preparations described in the above "6. Enzyme preparation" (enzyme preparation for measuring a ketone body), or (a2) dropping, placing, flowing, absorbing, or the like the sample on an insoluble support on which the active ingredient (modified HBDH) is immobilized in the sensor for measuring a ketone body described in the above "7. Sensor for measuring ketone body", or immersing the insoluble support in the sample.

The coexisting system of the sample and the modified HBDH may further contain the coenzyme, the reduction reagent, the redox mediator, and/or the buffer solution described in the above "8. Kit for measuring ketone body". The amount of the coenzyme used per 1 g of the modified HBDH is, for example, 0.01 to 100 mol, preferably 0.1 to 10 mol, more preferably 0.1 to 10 mol, further preferably 0.5 to 5.0 mol, and still more preferably 0.8 to 3.0 mol. The amount of the reduction reagent used per 1 g of the modified HBDH is, for example, 0.01 to 100 mol, preferably 0.1 to 50 mol, more preferably 0.1 to 10 mol, further preferably 0.5 to 5.0 mol, and still more preferably 0.8 to 3.0 mol. The amount of the redox mediator used per 1 g of the modified HBDH is, for example, 0.01 to 100 mol, preferably 0.1 to 50 mol, more preferably 0.1 to 10 mol, further preferably 0.5 to 5.0 mol, and still more preferably 0.8 to 3.0 mol.

In the step (b), as the reaction conditions, the temperature and pH conditions that cause the 3-hydroxybutyrate dehydrogenase activity of the modified HBDH are appropriately selected by those skilled in the art. For example, the reaction temperature is specifically, for example, 5 to 80°C, preferably 10 to 70°C, more preferably 15 to 60°C, further preferably 20 to 55°C, even further preferably 25 to 50°C, and still more preferably 30 to 50°C. The reaction pH can be based on the optimum pH of modified HBDH, and is specifically 3 to 9, preferably 5 to 8, and more preferably 6 to 7.5. The reaction time is not particularly limited, and is, for example, 10 seconds to 12 hours, preferably 30 seconds to 1 hour, and more preferably 1 minute to 30 minutes. Optimal conditions for these reaction conditions can be determined through a preliminary experiment.

Note that the step (a) and the step (b) may be performed in this order or may be performed simultaneously.

In the step (c), the derivative of $NAD^+$ is not particularly limited as long as it is a substance having $NAD^+$ as a basic skeleton and producing a reduced form at the same time as when 3-hydroxybutyric acid is converted into acetoacetic acid by the modified HBDH similarly to $NAD^+$, and examples thereof include nicotinamide adenine dinucleotide phosphate, thionicotinamide-NAD, pyridine aldehyde-NAD, acetylpyridine-NAD, and carba-NAD.

The change based on the reaction of 3-hydroxybutyric acid with $NAD^+$ and/or a derivative thereof is as described in the above "7. Sensor for measuring ketone body". The change based on the reaction of 3-hydroxybutyric acid with $NAD^+$ and/or a derivative thereof correlates with the amount of 3-hydroxybutyric acid contained in the sample. Therefore, 3-hydroxybutyric acid contained in the sample can be detected or quantified by detecting or quantifying the change based on the reaction.

In a preferred aspect, the amount of generated NADH and/or a derivative thereof; the amount of consumed $NAD^+$ and/or derivative thereof; and/or the ratio of these amounts can be measured as the amount of change based on the reaction on the basis of the correlation between the amount of generated NADH and/or a derivative thereof and the amount of consumed $NAD^+$ and/or a derivative thereof and the amount of 3-hydroxybutyric acid present in the sample. Methods for calculating these amounts and ratios can be appropriately determined by those skilled in the art. Examples

Hereinafter, the present invention will be specifically described by means of Examples; however, the present invention is not to be construed as being limited to the following Examples.

[Screening of modified HBDH]

Genomic DNA was extracted from Ralstonia pickettii. Using the gene sequence (SEQ ID NO: 13) of 3-hydroxybutyrate dehydrogenase (HBDH) as a template, saturated mutation introduction was performed to obtain a mutant gene product group. Each of the obtained mutant gene products was introduced into pTrc99A, and E.coli JM109 was transformed by a conventional method. The obtained transformant was cultured with shaking in LB-Lennox medium (1% Bacto Trypton: Thermo Fisher Scientific, 0.5% Bacto Yeast Extract: Thermo Fisher Scientific, 0.5% sodium chloride: Fujifilm Wako Co., Ltd.) at 37°C for 16 hours. Thereafter, the transformant was transplanted to Terrific Broth (1.2% Bacto Trypton: Thermo Fisher Scientific, 2.4% Bacto Yeast Extract: Thermo Fisher Scientific, 0.8% glycerol: Fujifilm Wako Co., Ltd., 0.94% dipotassium hydrogen phosphate: Fujifilm Wako Co., Ltd., 0.22% potassium dihydrogen phosphate: Fujifilm Wako Co., Ltd.) and cultured with shaking at 37°C for 20 hours. The bacterial cells were recovered from the culture broth, and the bacterial cells were crushed by a conventional method using Beads Shocker (Yasui Kikai Corporation) and

centrifuged at 15000 rpm for 10 minutes. The collected centrifugal supernatant was used as a crude enzyme solution.

The crude enzyme solution was subjected to treatment at 40°C for 15 minutes, and then HBDH activity was measured to calculate residual activity. The variants evaluated were 1500 strains, but only 11 strains in which the heat resistance effect could be confirmed were obtained as compared with the wild type. The strain confirmed to have heat resistance effect was subjected to sequence analysis to confirm a substituted amino acid. As a result, the amino acid sequences of SEQ ID NOS: 2 to 12 were identified. The crude enzyme solution of the strain confirmed to have heat resistance was purified by a conventional method such as adsorption chromatography or ultrafiltration to obtain a purified enzyme sample of modified HBDH having each of the amino acid sequences of SEQ ID NOS: 2 to 12.

[Test Example 1: Heat resistance evaluation-1 of modified HBDH]

Comparative Example 1 (purified enzyme sample of wild-type HBDH) and Examples 1 to 5 (purified enzyme sample of modified HBDH) in Table 2a were subjected to the following heat resistance evaluation.

The protein concentration of the prepared purified enzyme sample was measured using Biorad Protein assay reagent (Bio-Rad Laboratories, Inc.). The purified enzyme sample was diluted with 50 mM phosphate buffer solution (pH 7.5) to 0.1 mg/ml, and the protein concentrations of the purified enzyme samples were equalized. Each 100 μL was placed in a PCR tube (BM Equipment Co., Ltd.) and heat-treated with a thermal cycler (Applied Biosystems) at a temperature of 40°C, 45°C, 50°C, 55°C, or 60°C for 15 minutes. Thereafter, the mixture was rapidly cooled on ice, and the HBDH activity of the obtained sample solution was measured as follows.

To a 0.1 mol/L Tris-HCl buffer solution (pH 8.5) containing 145 mmol/L (final concentration) of 3-hydroxybutyric acid, 5.8 mmol/L (final concentration) of hydrazine sulfate, 0.1 g/dL (final concentration) of TritonX-100, and 13 mmol/L (final concentration) of β-NAD, 10 μL of a solution obtained by diluting the sample solution (0.1 mg/mL) at a predetermined magnification at 25°C was added, and then a change in absorbance at 340 nm was measured. The HBDH activity was measured on the basis of the following formula.

HBDH activity = (Absorbance A340 nm increasing per minute) × (1/6.22) × Dilution factor
6.22: Millimolar extinction coefficient of NADH at 340 nm
[Chemical Formula 2]

The HBDH relative activity (%) of each of Examples when the HBDH activity of Comparative Example 1 after the treatment at each temperature is taken as 100% is shown in Table 2a. The HBDH relative activity (%) after the treatment at each temperature when the HBDH activity at 4°C is taken as 100% is shown in Table 2b.

[Table 2a]

| Relative activity (%) when activity of Comparative Example 1 after treatment at each temperature is taken as 100% | | | | | |
|---|---|---|---|---|---|
| | | 40°C | 45°C | 50°C | 55°C |
| Comparative Example 1 | SEQ ID NO: 1 | 100 | 100 | 100 | 100 |
| Example 1 | SEQ ID NO: 3 | 131 | 129 | 362 | 244 |
| Example 2 | SEQ ID NO: 12 | 163 | | 777 | 1698 |
| Example 3 | SEQ ID NO: 2 | 117 | | | |
| Example 4 | SEQ ID NO: 11 | 111 | | | |
| Example 5 | SEQ ID NO: 7 | 107 | | | |

[Table 2b]

| Relative activity (%) after treatment at each temperature when activity at 4°C is taken as 100% | | | | | | | |
|---|---|---|---|---|---|---|---|
| | | 4°C | 40°C | 45°C | 50°C | 55°C | 60°C |
| Comparative Example 1 | SEQ ID NO: 1 | 100 | 101 | 103 | 16 | 2 | 0 |
| Example 1 | SEQ ID NO: 3 | 100 | 104 | 104 | 46 | 4 | 1 |
| Example 2 | SEQ ID NO: 12 | 100 | 111 | | 84 | 23 | 3 |
| Example 3 | SEQ ID NO: 2 | 100 | 117 | | | | |

[Test Example 2: Heat resistance evaluation-2 of modified HBDH]

Comparative Example 1 (purified enzyme sample of wild-type HBDH) and Examples 4 to 11 (purified enzyme sample of modified HBDH) in Table 3a were subjected to the following heat resistance evaluation.

The protein concentration of the prepared purified enzyme sample was measured using Biorad Protein assay reagent (Bio-Rad Laboratories, Inc.). The purified enzyme sample was diluted with 50 mM phosphate buffer solution (pH 7.5) to 0.1 mg/ml, and the protein concentrations of the purified enzyme samples were equalized. Each 100 μL was placed in a PCR tube (BM Equipment Co., Ltd.) and heat-treated with a thermal cycler (Applied Biosystems) at a temperature of 40°C, 45°C, 50°C, 55°C, or 60°C for 15 minutes. Thereafter, the mixture was rapidly cooled on ice, and the HBDH activity of the obtained sample solution was measured as follows.

A substrate solution composed of a 100 mmol/L PIPES-NaOH buffer solution (pH 7.0) containing 600 mmol/L of 3-hydroxybutyric acid, 60 mmol/L of β-NAD, 6.6 mmol/L of 1-methoxy-5-methylphenazinium methyl sulfur, 6.6 mmol/L of nitrotetrazolium blue chloride, and 0.1 g/dL (final concentration) of TritonX-100 was prepared. 10 μL of a sample solution diluted at a predetermined magnification was added to 200 μL of the substrate solution at 37°C, and then a change in absorbance at 570 nm was measured.

HBDH activity = (Absorbance A570 nm increasing per minute) × (1/4.02) × Dilution factor
4.02: Millimolar extinction coefficient of nitrotetrazolium blue chloride at 570 nm

[Chemical Formula 3]

The HBDH relative activity (%) of each of Examples when the HBDH activity of Comparative Example 1 after the treatment at each temperature is taken as 100% is shown in Table 3a. The HBDH relative activity (%) after the treatment at each temperature when the HBDH activity at 4°C is taken as 100% is shown in Table 3b.

[Table 3a]

| Relative activity (%) when activity of Comparative Example 1 after treatment at each temperature is taken as 100% | | | | | |
|---|---|---|---|---|---|
| | | 40°C | 45°C | 50°C | 55°C |
| Comparative Example 1 | SEQ ID NO: 1 | 100 | 100 | 100 | 100 |
| Example 4 | SEQ ID NO: 11 | 124 | 230 | 180 | 388 |
| Example 6 | SEQ ID NO: 4 | 205 | 409 | 939 | 1233 |
| Example 7 | SEQ ID NO: 10 | 119 | 238 | 102 | 283 |
| Example 8 | SEQ ID NO: 8 | 171 | 222 | 202 | 398 |
| Example 5 | SEQ ID NO: 7 | 105 | 178 | | |
| Example 9 | SEQ ID NO: 5 | | 117 | 430 | 350 |
| Example 10 | SEQ ID NO: 9 | | 184 | 714 | 424 |
| Example 11 | SEQ ID NO: 6 | | | 283 | 217 |

[Table 3b]

| Relative activity (%) after treatment at each temperature when activity at 4°C is taken as 100% | | | | |
|---|---|---|---|---|
| | | 4°C | 55°C | 60°C |
| Comparative Example 1 | SEQ ID NO: 1 | 100 | 2 | 0 |
| Example 4 | SEQ ID NO: 11 | 100 | 6 | 3 |
| Example 6 | SEQ ID NO: 4 | 100 | 13 | 2 |
| Example 7 | SEQ ID NO: 10 | 100 | 4 | |
| Example 8 | SEQ ID NO: 8 | 100 | 4 | 1 |
| Example 9 | SEQ ID NO: 5 | 100 | 10 | |
| Example 10 | SEQ ID NO: 9 | 100 | 7 | |
| Example 11 | SEQ ID NO: 6 | 100 | 9 | |

## Claims

1. A modified enzyme comprising a polypeptide shown in any one of the following (I) to (III):

   (I) a polypeptide consisting of an amino acid sequence obtained by introducing at least one of

   [A] a substitution of the amino acid residue at position 47 with a methionine residue,
   [B] a substitution of the amino acid residue at position 48 with a histidine residue,
   [C] a substitution of the amino acid residue at position 49 with a leucine residue,
   [D] a substitution of the amino acid residue at position 53 with a phenylalanine residue, a leucine residue, a proline residue, or a glutamic acid residue,
   [E] a substitution of the amino acid residue at position 54 with a leucine residue, an alanine residue, or a phenylalanine residue,
   [F] a substitution of the amino acid residue at position 55 with a glycine residue, a glutamic acid residue, a methionine residue, or a phenylalanine residue,
   [G] a substitution of the amino acid residue at position 63 with a leucine residue,
   [H] a substitution of the amino acid residue at position 81 with an isoleucine residue,
   [I] a substitution of the amino acid residue at position 147 with a glycine residue,
   [J] a substitution of the amino acid residue at position 210 with a valine residue,
   [K] a substitution of the amino acid residue at position 240 with an alanine residue,
   [L] a substitution of the amino acid residue at position 241 with a phenylalanine residue,
   [M] a substitution of the amino acid residue at position 250 with a valine residue,
   [N] a substitution of the amino acid residue at position 251 with a proline residue, and
   [O] a substitution of the amino acid residue at position 252 with an isoleucine residue,

   into the amino acid sequence set forth as SEQ ID NO: 1;
   (II) a polypeptide in which one or several amino acid residues other than the substituted amino acid residues are substituted, added, inserted, or deleted in the amino acid sequence in which the substitution shown in at least one of [A] to [O] is introduced, and the 3-hydroxybutyrate dehydrogenase activity after treatment at least at one of prescribed temperatures in a temperature range of 40°C or higher is improved as compared to the activity of the polypeptide consisting of the amino acid sequence set forth as SEQ ID NO: 1 after treatment at the prescribed temperature; and
   (III) a polypeptide in which the sequence identity of regions that do not include the substituted amino acid residues is 70% or more in the amino acid sequence in which the substitution shown in at least one of [A] to [O] is introduced, and the 3-hydroxybutyrate dehydrogenase activity after treatment at least at one of prescribed temperatures in a temperature range of 40°C or higher is improved as compared to the activity of the polypeptide consisting of the amino acid sequence set forth as SEQ ID NO: 1 after treatment at the prescribed temperature.

2. A DNA encoding the modified enzyme according to claim 1.

3. An expression cassette or recombinant vector comprising the DNA according to claim 2.

4. A transformant obtained by transforming a host using the expression cassette or recombinant vector according to claim 3.

5. A method for producing a modified enzyme, the method comprising a step of culturing the transformant according to claim 4.

6. An enzyme preparation comprising the modified enzyme according to claim 1.

7. A sensor for measuring a ketone body, comprising the modified enzyme according to claim 1.

8. A kit for measuring a ketone body, comprising the modified enzyme according to claim 1.

9. A method for measuring a ketone body in a sample, comprising a step of bringing a sample for which a ketone body is to be measured into contact with the modified enzyme under the modified enzyme activity condition according to claim 1.

**INTERNATIONAL SEARCH REPORT**

International application No.
**PCT/JP2024/040387**

**A. CLASSIFICATION OF SUBJECT MATTER**

***C12N 9/04***(2006.01)i; ***C12N 1/15***(2006.01)i; ***C12N 1/19***(2006.01)i; ***C12N 1/21***(2006.01)i; ***C12N 5/10***(2006.01)i; ***C12N 15/53***(2006.01)i; ***C12N 15/63***(2006.01)i; ***C12Q 1/32***(2006.01)i
FI: C12N9/04 Z ZNA; C12N1/15; C12N1/19; C12N1/21; C12N5/10; C12N15/53; C12N15/63 Z; C12Q1/32

According to International Patent Classification (IPC) or to both national classification and IPC

**B. FIELDS SEARCHED**

Minimum documentation searched (classification system followed by classification symbols)

C12N9/04; C12N1/15; C12N1/19; C12N1/21; C12N5/10; C12N15/53; C12N15/63; C12Q1/32

Documentation searched other than minimum documentation to the extent that such documents are included in the fields searched

Published examined utility model applications of Japan 1922-1996
Published unexamined utility model applications of Japan 1971-2025
Registered utility model specifications of Japan 1996-2025
Published registered utility model applications of Japan 1994-2025

Electronic data base consulted during the international search (name of data base and, where practicable, search terms used)

JSTPlus/JMEDPlus/JST7580 (JDreamIII); CAplus/MEDLINE/EMBASE/BIOSIS (STN); UniProt/GeneSeq

**C. DOCUMENTS CONSIDERED TO BE RELEVANT**

| Category* | Citation of document, with indication, where appropriate, of the relevant passages | Relevant to claim No. |
|---|---|---|
| A | WO 2021/210614 A1 (AMANO ENZYME INC.) 21 October 2021 (2021-10-21) claims, examples | 1-9 |
| A | JP 2019-511245 A (F. HOFFMANN-LA ROCHE AG) 25 April 2019 (2019-04-25) claims, examples | 1-9 |
| A | JP 2019-505193 A (F. HOFFMANN-LA ROCHE AG) 28 February 2019 (2019-02-28) claims, examples | 1-9 |
| A | WO 2021/149675 A1 (TOYOBO CO., LTD.) 29 July 2021 (2021-07-29) claims, examples | 1-9 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 January 2025** | **14 January 2025** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/040387**

**C. DOCUMENTS CONSIDERED TO BE RELEVANT**

| Category* | Citation of document, with indication, where appropriate, of the relevant passages | Relevant to claim No. |
|---|---|---|
| A | GROSS, H. et al. Accession No. A0A2P4RL44. Definition: SubName: Full=3-hydroxybutyrate dehydrogenase {ECO:0000313\|EMBL:POH89011.1}; DE EC=1.1.1.30 {ECO:0000313\|EMBL:MBB0172030.1};. Database UniProt/GeneSeq [online]. 2018, internet <URL: https://rest.uniprot.org/uniprotkb/A0A2P4RL44.txt>. 23 May 2018 uploaded [retrieved on 06 January 2025] in particular, columns AC, DE, OS, SQ | 1-9 |
| A | TAKANASHI, M. et al. Characterization of two 3-hydroxybutyrate dehydrogenases in poly (3-hydroxybutyrate)-degradable bacterium, Ralstonia pickettii T1. JOURNAL OF BIOSCIENCE AND BIOENGINEERING. 2006, vol. 101, no. 6, pp. 501-507, ISSN:1389-1723 in particular, abstract | 1-9 |

Form PCT/ISA/210 (second sheet) (July 2022)

**INTERNATIONAL SEARCH REPORT**

International application No.
**PCT/JP2024/040387**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/JP2024/040387**

| Patent document cited in search report | Publication date (day/month/year) | Patent family member(s) | Publication date (day/month/year) |
|---|---|---|---|
| WO 2021/210614 A1 | 21 October 2021 | (Family: none) | |
| JP 2019-511245 A | 25 April 2019 | US 2018/0346886 A1<br>claims, examples<br>EP 3414325 A1<br>WO 2017/137491 A1<br>CN 108473966 A | |
| JP 2019-505193 A | 28 February 2019 | US 2018/0291354 A1<br>claims, examples<br>EP 3394252 A1<br>WO 2017/109003 A1<br>CN 108350439 A<br>KR 10-2018-0087329 A | |
| WO 2021/149675 A1 | 29 July 2021 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H11318438 A
- JP H870856 A
- JP 2003339385 A


### Non-patent literature cited in the description


- *Biochim. Biophys. Acta*, 1986, vol. 871, 302-309
- *J. Biosci. Bioeng*, 2006, vol. 101, 501-507
- *J. Gen. Microbiol*, 1978, vol. 104, 123-126
- *Acta Microbiol. Pol.*, 1994, vol. 43, 33-45
- *J. Mol. Biol.*, 2006, vol. 355, 722-733
- *Acta Crystallogr. Sect. F*, 2009, vol. 65, 331-335
- *J. Biosci. Bioeng.*, 2004, vol. 97, 78-81
- *Can. J. Microbiol.*, 1973, vol. 19, 673-677
- *J. Biochem.*, 1981, vol. 89, 625-635
- FEMS Microbiol. Lett. **TATIANA A. TATSUSOVA** ; **THOMAS L. MADDEN**. BLAST PACKAGE. National Center for Biotechnology Information (NCBI), 1999, vol. 174, 247-250
- **SANGER et al.** *Proc. Natl. Acad. Sci. USA*, 1977, vol. 74, 5463